(19) Europäisches Patentamt
European Patent Office
Office européen des brevets

(11) EP 0 476 455 B1

(12) **EUROPEAN PATENT SPECIFICATION**

(45) Date of publication and mention of the grant of the patent:
**04.11.1998 Bulletin 1998/45**

(21) Application number: **91115072.0**

(22) Date of filing: **06.09.1991**

(51) Int. Cl.$^6$: **C07D 417/04**, C07D 417/14, C12Q 1/26, C12Q 1/00, G01N 33/52
// (C07D417/04, 277:00, 257:00), (C07D417/14, 333:00, 277:00, 257:00), (C07D417/14, 317:00, 277:00, 257:00), (C07D417/14, 277:00, 257:00, 213:00), (C07D417/14, 277:00, 257:00, 215:00)

(54) **Phenyl-substituted 2-thiazolyl tetrazolium salt indicators**

Phenylsubstituierte 2-Thiazolyl-tetrazolium-Salze als Indikatoren

Sels de 2-thiazolyl-tétrazolium substitués par de phényle, comme indicateurs

(84) Designated Contracting States:
**AT BE CH DE DK ES FR GB GR IT LI LU NL SE**

(30) Priority: **19.09.1990 US 585725**

(43) Date of publication of application:
**25.03.1992 Bulletin 1992/13**

(73) Proprietor: **Bayer Corporation**
**Pittsburgh, PA 15205-9741 (US)**

(72) Inventors:
- **Köcher, Jürgen, Dr.**
  **W-5000 Koeln 80 (DE)**
- **Wehling, Klaus, Dr.**
  **W-5600 Wuppertal 1 (DE)**

(74) Representative:
**Dänner, Klaus, Dr. et al**
**Bayer AG**
**Konzernbereich RP**
**Rechtspolitik Gewerblicher Rechtsschutz**
**51368 Leverkusen (DE)**

(56) References cited:
**EP-A- 190 740** **EP-A- 239 931**
**DE-A- 3 247 894**

- **DIE PHARMAZIE, vol. 34, no. 12, 1979, BERLIN, DD pages 790 - 794; S. JOHNE ET AL.: 'Darstellung einiger neuer ...'**
- **PATENT ABSTRACTS OF JAPAN vol. 9, no. 211 (C-300)(1934) 29 August 1985 & JP-A-60 075 470 ( WAKO JUNYAKU KOGYO K.K. ) 27 April 1985**
- **CHEMICAL ABSTRACTS, vol. 95, no. 19, 9 November 1981, Columbus, Ohio, US; abstract no. 169190J, page 743 ; & JP-A-56 061 367 (DOJINDO LABORATORIES), 26-05-81**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 476 455 B1

## Description

BACKGROUND OF THE INVENTION

The present invention relates to chromogenic tetrazolium salt indicator compounds useful in the determination of reducing substances, particularly nicotinamide adenine dinucleotide (NADH).

Tetrazolium salts are well known as chromogenic indicators responsive to reducing substances. Upon reduction, tetrazolium salts are converted into formazan dye products. These indicators have found use in a wide variety of fields, particularly the medical diagnostic field where they have been applied to, among others, cell staining and the determination of analytes in body fluids such as urine, milk, serum, and plasma. Commonly, the determination of body fluid analytes involves an NAD-dependent enzymatic reaction in which NADH is formed as a function of the amount of analyte present in the sample tested. The amount of NADH generated can then be determined by the reductive conversion of an appropriate tetrazolium salt indicator to its formazan dye product.

Within the field of medical diagnostic tests, tetrazolium salt indicators are useful in a variety of different product types. One particular type is the reagent strip. This product is a solid state device comprising a paper or other porous carrier matrix which is impregnated or otherwise incorporated with chemical reagents responsive to a particular analyte, for example, glucose or cholesterol. The incorporated reagent system includes a chromogenic indicator which develops color, or changes color, as a function of the amount of analyte in a sample applied to the matrix. The resulting colorimetric response can be observed visually to give qualitative or semi-quantitative readings. Quantitative results can be obtained by reading the reflectance of the matrix surface at one or more defined wavelengths with an appropriate instrument (reflectance meter).

There is a recognized need to develop tetrazolium indicators having strong absorbance at wavelengths longer than the absorbances of major interferants that can be present in the test sample. For instance, interference from hemoglobin coloration is a particular concern where the sample is whole blood. Indicators having significant absorption above about 640 nm are required in order to substantially overcome hemoglobin interference. The commonly used tetrazolium salt indicators are 2-(4-iodophenyl)-3-(4-nitrophenyl)-5-phenyltetrazolium chloride (INT), 3-(4,5-dimethylthiazol-2-yl)-2,5-diphenyltetrazolium chloride (MTT), and 2,2',5,5'-tetraphenyl-3,3'-(3,3'-dimethoxy-4,4'-diphenylene) ditetrazolium chloride (NBT). These compounds show maximum absorption (UVmax) in the range of 465 - 605 nm.

Another shortcoming of the conventionally used prior art tetrazolium salt indicators relates to the evolution of the instrumentation used to measure their colorimetric response. Rapid advancements are being made in developing smaller, less expensive reflectance meters. One of the more costly components of such meters is the optical system which comprises a light source, a filter or other spectral element for selecting or limiting the wavelength of incident or reflect light, and a sensor. Significant cost savings could be realized by eliminating or combining functions of the optical system elements or by using less expensive components, e.g., LEDs as illuminating light sources. However, commercially available LEDs emit light having a center wavelength that can vary signaficantly due to manufacturing variances and temperature dependence. The conventionally used tetrazolium salt indicators INT, MTT, and NBT have reflectance spectra which are strongly sloped in the region above their UVmax. Accordingly, without individually calibrating both each instrument, to account for manufacturing variability in the LED, and each test run, to account for variance due to temperature, large errors can be introduced to the assay result.

The following are representative of the prior art teachings concerning the use of various tetrazolium salts in colorimetric analysis. Tanaka et al, Japanese Kokai Tokkyo Koho JP 61000084 (Chem. Abst. 104:203469y) describes the detection of glucose using a formazan chelate obtained by the reduction of 2-(2-benzothiazolyl)-3-(carboxyphenyl) -5-phenyl-2H-tetrazolium halide in the presence of nickel (II). Limbach et al, German DE 3,247,894 (Chem. Abst. 101:125929v) relates to the use of INT in glucose assays. Rittersdorf et al, German DE 2,147,466 describes the use of seven 2-(2-benzothiazolyl)-3-phenyl-5-(4-[trimethylammonio]phenyl) tetrazolium salts in the determination of reducing substances such as reducing sugars, ascorbic acid, and ketosteroids.

The variety of 2-thiazolyl tetrazolium salts and/or their corresponding formazans known in the literature are represented by the following. Serebryakova et al, Khim. Geterotsikl. Soedin. 10:1403-1405 (1970) describe the synthesis and chromatic properties of benzothiazolyl-3-phenyl(methyl)-5-p-nitro(dimethylamino)phenylformazans. The authors state that both an electron-withdrawing nitro group at the para-position of the 5-phenyl and a benzothiazolyl group at the 1-position provides a bathochromic shift. Lipunova et al, Khim. Geterotsikl. Soedin. (1971) 831-835 compare the bathochromic effect of a 5-naphthyl or o-tolyl group on the visible spectrum of 1-benzothiazolyl-formazans. Johne et al, Pharmazie 34:790-794 (1979) describe certain 2-(4,5-diphenyl)thiazol-2-yl tetrazolium salts.

EP 239 931 describes an enzyme assay using certain tetrazolium salts.

EP 190 740 deals with the use of substituted quinon electron transfer agents in analytical determinations.

DE 32 47 894 is directed to a test system for the determination of NAD(P)H.

## SUMMARY OF THE INVENTION

The present invention provides thiazolyl tetrazolium salts which upon reduction yield formazans having new and improved optical properties. The compound of the present invention are phenyl substituted 2-thiazolyl tetrazolium salt indicators characterized by the formula:

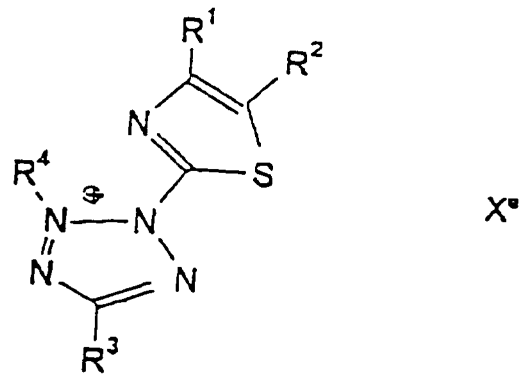

wherein $R^1$ and $R^2$ are such that the 2-thiazolyl group is 4,5-bis(4-methoxyphenyl)thiazol-2-yl, $R^4$ is 4-carboxyphenyl and $R^3$ is 2-thienyl and $X^{\ominus}$ is a counterion, and wherein the compounds are further characterized in that the reflectance spectra exhibited by each of said compounds when reduced to its colored formazan state varies by less than about 17% over a wavelength range of 50 nm when the midpoint of the 50 nm range is 640 nm or greater.

2. Phenyl substituted 2-thiazolyl tetrazolium salt indicators characterized by the formula:

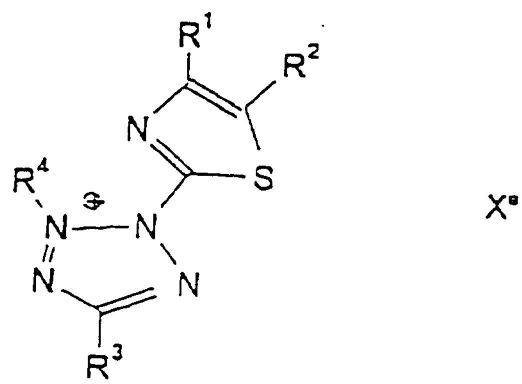

wherein $R^1$ and $R^2$ are such that the 2-thiazolyl group is 4-phenylthiazol-2-yl; $R^4$ is 4-carboxyphenyl and $R^3$ is 3,4-methylenedioxyphenyl or $R^1$ and $R^2$ are such that the 2-thiazolyl group is 4-(p-fluorophenyl)thiazol-2-yl; $R^4$ is 4-carboxyphenyl and $R^3$ is 3,4-methylenedioxyphenyl or $R^1$ and $R^2$ are such that the 2-thiazolyl group is 4-phenyl-5-methylthiazol-2-yl; $R^4$ is 4-carboxyphenyl and $R^3$ is 2-thienyl or 4-methylphenyl or $R^1$ and $R^2$ are such that the 2-thiazolyl group is 4-naphthyl-5-phenylthiazol-2-yl; $R^4$ is 4-carboxyphenyl and $R^3$ is 3,4-methylenedioxyphenyl or 2-thienyl and $X^{\ominus}$ is a counterion and wherein the compounds are further characterized in that the reflectance spectra exhibited by each of said compounds when reduced to its colored formazan state varies by less than about 17% over a wavelength range of 50 nm when the midpoint of the 50 nm range is 640 nm or greater.

## BRIEF DESCRIPTION OF THE DRAWINGS

Figs. 1 - 4 show the reflectance spectra of the formazans produced upon reduction of the prior art tetrazolium salts INT, MTT, and NBT at various concentrations of glucose.

Figs. 5 shows the corresponding spectrum for the formazan from the present compound 2-(4,5-bis(4-methoxyphenyl)-thiazol-2-yl)-3-(4-carboxyphenyl)-5-(2-thienyl) tetrazolium salt.

DESCRIPTION OF THE PREFERRED EMBODIMENTS

The present invention concerns 2-(4,5-Bis(4-methoxyphenyl) thiazol-2-yl)-3(4-carboxyphenyl)-5-(2-tienyl) tetrazolium salt.

The present invention further concerns the use of the above compounds 3 for the detection of a reducing substance, preferably NADH and reagent for determination of a reducing substance comprising one or more of these compounds.

Counteranion

The selection of the counteranion will be based primarily on considerations of stability and solubility of the particular tetrazolium salt of interest. In general, one can select from such counteranions as the inorganic anions chloride, bromide, iodide, nitrate, fluroborate, perchlorate, and sulfate, as well as organic anions such as acetate, oxalate, tartrate, and aryl sulfonates (benzene sulfonate, tosylate).

Synthetic Methods

Tetrazolium salts are prepared by methods well known in the literature (Hooper, W.D., Rev. Pure and Appl. Chem., 1969, 19, 221; Putter, R., in Methoden der Organischen Chemie, Houben-Weyl-Muller ed., Thieme Verlag: Stuttgart, 1965, Bd. 10/3, p. 633; Nineham, A. W. Chem. Rev., 1955, pp. 355-483). In general, the tetrazolium salts of the present invention are prepared by first reacting a 2-hydrazinothiazole with an aldehyde and then treating the resulting hydrazone with a diazotized aniline. The resulting formazan is then oxidized to the tetrazolium salt by well known methods. Consequently, the synthesis involves three principal starting materials, the aldehyde, the aniline, and the 2-hydrazinothiazole.

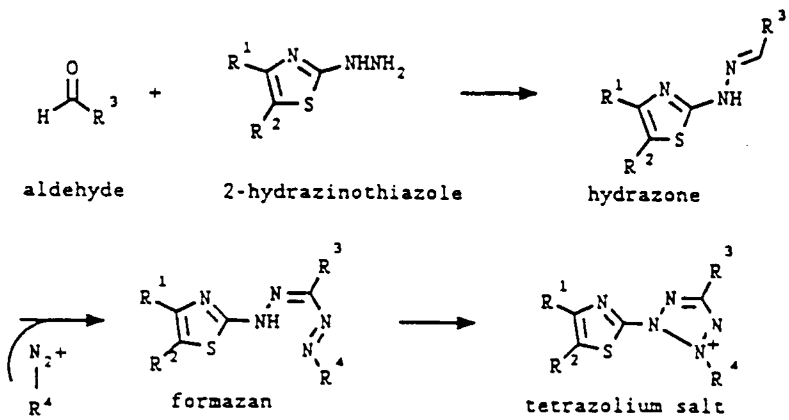

aldehyde 2-hydrazinothiazole hydrazone

formazan tetrazolium salt

diazotized aniline

Preparation of 2-hydrazinothiazoles

4,5-Diphenyl-2-hydrazinothiazoles are prepared by two methods. One is by first forming a benzoin condensation (1) product between two aldehydes (Ide, W.S., Buck, J.S., Org. React., 1948, Vol. 4, 269). Benzoins not available by this method are prepared from the condensation of a phenyl substituted "acyl anion equivalent" with another aldehyde followed by removal of the carbonyl protecting group (Bertz, S., J. Chem. Soc., Chem. Comm., 1980, 17, 831). A specific example is the condensation of the metallated O-trimethylsilylcyanohydrin (3) with an aryl aldehyde. The product (4) is deprotected using aqueous acetic acid to afford the benzoin product (1).

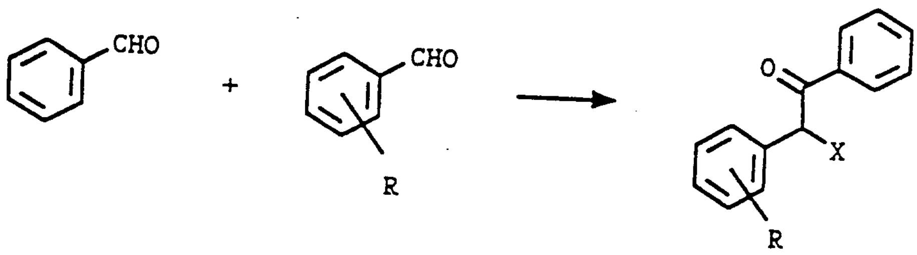

1. X = OH
2. X = Cl, Br, I

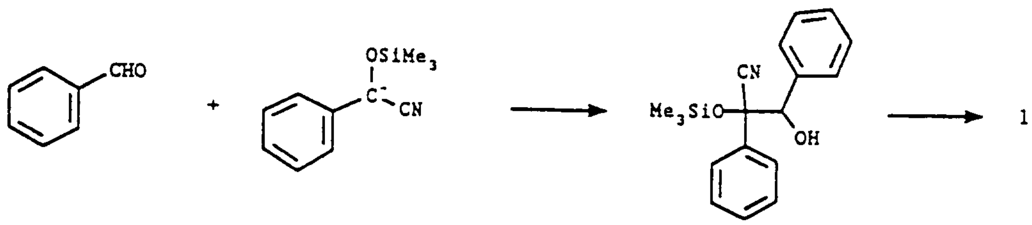

1

3

4

The benzoin products are then converted into the halide (2) using conventional reagents such as thionyl chloride to produce the α-haloketones (2) which react with thiourea to produce 4,5-diphenyl-2-aminothiazoles (Traumann, V., Liebigs Ann. Chem., 1888, 250, 31, Dodson et al., J. Am. Chem. Soc., 1945, 67, 2442). These can be converted to the 2-hydrazino compounds with hydrazine as described for the benzo examples.

The same α-haloketones react with thiocyanate to produce α-thiocyanoketones (5) which readily cyclize. For instance, when treated with hydrogen chloride gas, the 2-chlorothiazoles (6) are obtained which react with hydrazine to give the 2-hydrazinothiazoles.

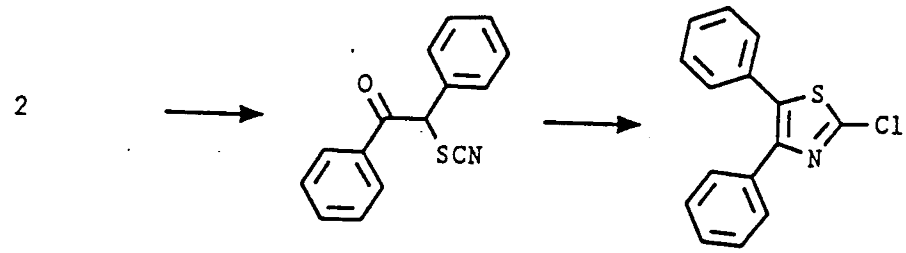

2

5

6

Alternatively, the α-haloketones may be used to directly yield the necessary hydrazone (8) by treating the haloketone with an N-arylthiosemicarbazone (7) (Johne, S., Schaks, A., Hartung, S., Scharf, K.-D., and Nover, L., Pharmazie, 1979, 34, 790).

2 +

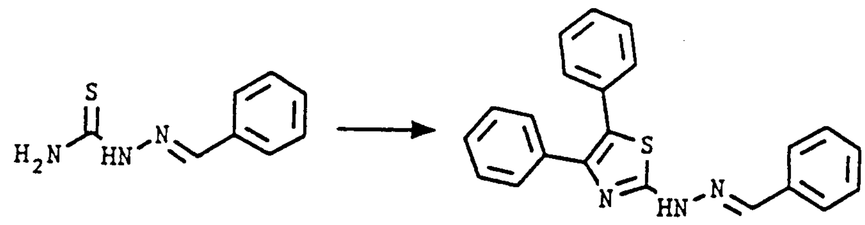

7

8

Thiazoles substituted in the 4-position with aryl and the 5-position with alkyl or hydrogen may be prepared similarly with a $\alpha$-haloketone and thiosemicarbazone or thiourea.

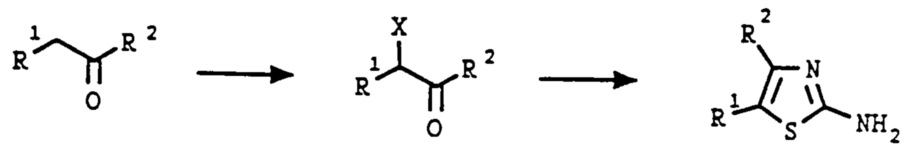

$R^1$ = alkyl, hydrogen
$R^2$ = aryl

Preparation of aldehydes

The aldehydes are obtained from commercial sources or can be prepared by methods familiar to one of ordinary skill in the art.

For instance, aldehydes may be prepared by benzylic oxidation of an arylmethane (March, J., Advances Organic Chemistry Third Edition; John Wiley and Sons: New York, 1985; p. 1079), reduction of an aryl acid chloride (Ibid. p. 396) or aryl acid derivative, [Larock, R.C., Comprehensive Organic Transformations; VCH: New York, 1989; pp. 604-605).

Aryl halides may also be used to synthesize aldehydes. In this method, a transmetallation reaction produces an arylmetallic species which can be treated with a variety of reagents, such as dimethylformamide, to produce the aldehyde (ibid, p. 681-683).

The aforementioned aryl aldehydes, acids, methanes, and halides, may be derivatized with a variety of functional groups prior to their transformation into tetrazolium salts. This may be accomplished by aromatic nucleophilic substitution (March, J., Advanced Organic Chemistry Third Edition; John Wiley and Sons: New York, 1985; pp. 576-607), aromatic electrophilic substitution (ibid., pp. 447-511) or heteroatom-directed metallation reactions (Gschwend, H.W., Rodriguez, H.R., in Organic Reactions, John Wiley and Sons: New York, 1979; Vol. 26, 1).

In cases where the aldehyde piece of the tetrazolium salt contains a phenol or amine, the groups must be protected so that there is not a reaction between these and the diazotized aniline or oxidising reagent used to prepare the tetrazolium salt.

This can be accomplished by protecting a hydroxyaryl aldehyde as an acetate, performing the reaction sequence to make the formazan, and then hydrolyzing the acetate at pH 10. Acidification to pH 5 and then filtration produces the desired formazan.

Where the resulting phenol formazan reacts with oxidizing agent in the tetrazolium salt preparation, the phenol may be protected by an acid labile group such as dihydropyran (Greene, T.W., Protective Groups in Organic Synthesis, John Wiley and sons: New York; 1981, pp. 87-113) and then removed by stirring the tetrazolium salt in acidic conditions.

Similarly amines on the aldehyde piece must be protected to prevent their reaction. This is best accomplished by using an acid labile carbamate (ibid, pp. 218-247) which is later removed by stirring the tetrazolium salt under acidic conditions.

Preparation of aryl amines

Aryl amines may be prepared by reduction of the corresponding nitro or azide compound (Larock, P.C., Comprehensive Organic Transformations; VCH: New York, 1989; pp. 412-415 or 409-410), reaction between an arylmetallic compound and an electrophilic nitrogen reagent, (ibid., pp. 399-400), or rearrangement of acyl azides or oxidized amides (ibid., pp. 431-432).

As in the aldehyde case, electrophilic and nucleophilic aromatic substitution can be used to introduce different functional groups into the aryl amine or synthetic precursor.

Use of the Compounds

The principal use of the tetrazolium salt compounds of the present invention are as chromogenic indicators for detecting reducing substances. In particular, the present compounds are advantageous in the detection of NADH. As such, since NADH is produced in enzyme-catalyzed detection reactions specific for various biochemical substances, the present compounds are particularly useful in medical diagnostic tests. However, in general other reducing substances can also be detected, such as hydrogen sulfide gas, diborane, arsenic hydride, or phosphorus hydride.

The present compounds have been particularly found to exhibit an extended plateau in their reflectance spectrum above about 600 nm. The most preferred indicator compounds of the present invention have a plateau above about 650 nm (i.e., the flatest about 50 nm wide portion begins between 640 and 660 nm). Such a reflectance plateau confers improved accuracy to analytical tests based on the measurement of reflectance from a reagent strip.

Reagent strips are known in the art as analytical devices comprising a solid carrier matrix incorporated with a test composition that produces a color change in response to contact with a liquid test sample containing the analyte of interest. Such test composition, in the case of the present invention, comprises (a) a reagent or reagents which react with the analyte to produce a reducing substance, and (b) a 2-thiazolyl tetrazolium salt as described herein which is reducable by such reducing substance to produce a chromogenic formazan dye product. The color response of such reagent strips can be observed visually to give semi-quantitative values, however, quantitative results are obtained by measuring the reflectance of the carrier matrix at a predetermined wavelength. Such measurements involve irradiating the reacted carrier matrix with a light source and sensing the reflectance of the carrier matrix by measuring reflected light with a detector element.

The finding of tetrazolium salt indicators having a reflectance plateau is used to particular advantage where reflectance from a reagent strip is read using an instrument which is subject to variability in the central wavelength of its optical system (the combination of light source, detector element, spectral control elements, e.g., filters), and other components). Variability in the central wavelength of the optical system can be caused by a variety of factors, for example, variability in the central wavelength of the principal spectral control element such as the illuminating light source or filters. For instance, where light emitting diodes (LEDs) are used as the light source, the wavelength of emitted light will typically vary ± 4 nm within an instrument, and up to ± 8 nm between LEDs in different instruments, due to manufacturing variability. Moreover, LEDs are suceptible to variable central wavelength due to temperature effects as well. Where broad band light sources are used with filters to provide spectral control of the central wavelength, variability within an instrument is typically under 1 nm, however, between instrument variability can be as high as ± 6 nm. Thus, the present invention is applicable in those situations where the central wavelength of the light reaching the detector element in the instrument is susceptible to variations in the range of about ± 5 nm.

In the making of a reagent strip for use in the present invention, selection of the carrier matrix, the test reagents which react with analyte to produce the reducing substance, and the method by which such reagents and the tetrazolium indicator are incorporated with the carrier matrix are matters well known in the art of reagent strips. For the sake of reciting just a few examples, typical carrier matrices are porous and absorbent paper, cloth, glass fiber filters, polymeric membranes and films, and the like. Incorporation methods include impregation of a formed carrier matrix with a solution, suspension, or other liquid form of the test composition, in one or more steps, followed by drying of the matrix; formation of a matrix in the presence of one or more of the components of the test composition, e.g., by casting or layering solutions of film or membrane forming formulations.

The present invention will now be illustrated, but is not intended to be limited by, the following examples.

EXAMPLES

A. Compound Synthesis

Hydrazone Preparations

Preparation of aryl aldehyde 4,5-diarylthiazol-2-yl hydrazones and aryl aldehyde 4-aryl-5-alkyl or hydrogen thiazol-2-yl hydrazones

A mixture of 50 mmol of the thiosemicarbazone, 50 mmol of the α-chloroketone and 100 mmol of pyridine in 200 mL of ethanol was refluxed for one hour. The mixture was cooled to room temperature and then filtered to yield to hydrazone.

Preparation of aryl aldehyde 4-styryl-5-arylthiazol-2-yl hydrazones

Compounds of the type 1,4-diaryl-2-oxo-3-butene were prepared by the method of S. A. Fine and P. D. Pulaski in J. Org. Chem. 38, 1747 (1973). Following the procedure of P. L. Southwick and D. I. Sapper in J. Org. Chem. 1926 (1954), a mixture of 130 mmol of a 1,4-diaryl-2-oxo-3-butene, 130 mmol of iodine and 260 mmol of a thiosemicarbazone was refluxed in 1.2 L of ethanol for 5 hours. The mixture was cooled to room temperature and filtered to yield the hydrazone.

Formazan Preparation

The diazonium salt is first prepared by cooling a slurry or solution of 8.5 mmol of the amine in 60 mL of 3 N HCl to 5°C. Sodium nitrite (0.70 g, 10.15 mmol) in 5 mL of water is then added dropwise. After stirring for 30 minutes, the mixture added dropwise to a cold (-25°C) mixture of 8.5 mmol of the hydrazone in 120 mL of 1:1 (v/v) DMF-pyridine. The reaction is not allowed to warm beyond -15°C during the addition. The mixture is allowed to warm to room temperature while stirring for two hours. Filtration produces the formazan as a black solid. Impurities can be removed by repeated washing with methanol or refluxing the solid in methanol and filtering while hot.

Tetrazolium Salt Preparation

A slurry of 1.5 mmol of the formazan is stirred with 20 mL of acetic acid and 4 mL of isoamyl nitrite for a period of 16-48 hours. The mixture is then filtered to yield the tetrazolium salt. In cases where the salt does not precipitate, dilution with ether caused precipitation.

B. Preparation of Reagent Strips

Indicators were impregnated into a reagent strip and tested with a solution containing a known quantity of glucose or NADH. The reagent strip consists of a polystyrene handle onto which a single reagent pad is attached. The reagent pad was 0.2 x 0.2" square and contains reagents allowing for a color change which was instrumentally read when an aliquot of sample containing glucose was applied. The dry-phase reagent pad is a solid support composed of cellulosic fibers or a nylon membrane as examples. The reagent pad was impregnated first with a solution of the tetrazolium salt of interest (0.8M/L) and detergent (0.3%) in a solvent such as methanol. The second solution impregnated into the reagent pad contains the following components:

| | |
|---|---|
| Glucose Dehydrogenase (GDH) | 0.8U/L |
| Diaphorase (DPH) | 0.8U/L |
| NAD | 0.03 Mol/L |
| PIPES Buffer | 0.15 Mol/L |
| Detergent | 0.5% |

About 0.01 ml of several test solutions (serum, plasma, aqueous) containing at least five different glucose or NADH

concentrations between 0 and 33 mM/L was applied to the center of the dried reagent pad. After a lag time of about 60 seconds, the reflectance spectra of each indicator was measured at 5 nm increments over the wavelength range of 400 to 1100 nanometers.

C. Utility Data

Following is a table of spectral and other analytical data pertaining to various synthesized tetrazolium salts of the present invention. The compounds are organized, in order, by the form of their thiazolyl residue, then by their $R^4$ substituent and finally by their $R^3$ substituent. For example, the first compound presented is A.1.a) and is of the Formula A wherein $R^1$ and $R^2$ are both 4-methoxyphenyl, $R^4$ is 4-carboxyphenyl, and $R^3$ is 2-thienyl; the second compound, B.1.a), has a different thiazolyl residue ($R^1$ and $R^2$ are both unsubstituted diphenyl), $R^4$ is carboxyphenyl, and $R^3$ is 3,4-methylenedioxyphenyl; and so forth.

The reflectance spectrum of tetrazolium salts is understood to be dependent upon the environment in which they are observed or measured. For purposes of comparison between individual tetrazolium salts, the data below include a measurement of the relative flatness of the flatest portion of the reflectance spectrum at wavelengths greater than 600 nm, which spectrum is generated using a glucose or cholesterol reagent strip prepared as described in Part B above. The relative flatness of the spectrum is expressed in the data in K/S units normalized for the level of analyte detected as defined below.

K/S is defined by the equation

$$\frac{(1-R)^2}{2R}$$

wherein R is instrumentally read reflectance units. Percent change in K/S is the change, expressed as a percentage, over a 50 nm range divided by the average of the high and low K/S values over the range.

The plateau property of the present compounds shall be understood, for the purposes of this invention, as a percent change in reflectance spectrum (expressed in terms of K/S as defined in the paragraph above) of less than about 17% over a 30-50 nm wavelength span beginning at a wavelength above about 600 nm. The more preferable compounds exhibit a plateau having a percent change in K/S of less than an about 10% over a 50 nm wavelength span. Most preferred are those tetrazolium salt indicators exhibiting a percent change in K/S of about 5% or less over a 50 nm wavelength span. Compounds having a more sloped reflectance spectrum are nonetheless preferred where the flatest portion is a wavelength above 650 nm, preferably above 675 nm.

With reference to the drawings, Figs. 1-4 show the reflectance spectra of the formazans produced upon reduction of the prior art tetrazolium salts INT, MTT, and NBT at various concentrations of glucose. For purposes of comparison, Fig. 5 shows the corresponding spectrum for the present compound

(see item A-1-a in the Table below) when tested with various levels of NADH. The presence of a plateau in the spectra of the formazans from the present compounds, and its absence from that of the formazans from the prior art compounds, is readily apparent.

The four above-mentioned prior art compounds exhibit percent changes in K/S over the wavelength range 650-700 nm as follows:

| | |
|---|---|
| INT | 71% |
| MTT | 178% |
| NBT | 73% |
| USSR | 28% |

The lower the percent K/S value for the formazan, the more tolerant is the tetrazolium salt to variations in the central wavelength of the optical system used to measure reflectance, and hence to measure analyte concentration.

The following non-standard abbreviations are used in the text below:

"UV" - The wavelength in nanometers of maximum reflectance peak in the UV reflectance spectrum of the formazan. The extinction coefficient and solvent used during measurement are given in parentheses.

"nm" - The position of the flatest portion of the reflectance spectrum of the formazan over a 50 nm wavelength span (expressed as the beginning and ending wavelengths in nanometers).

"K/S" The percent change in K/S units over the above mentioned flatest 50 nm portion of the reflectance spectrum.

The concentration of analyte used to generate the reflectance spectrum is given in parentheses. Mmol refers to the concentration in mmol/liter.

TABLE

A. 4,5-bis(4-methoxyphenyl)thiazol-2-yl

1. $R^4$ = 4-carboxyphenyl

a) $R^3$ = 2-thienyl
UV: 623 (11.6 x $10^3$, water)
nm: 660 - 710 nm; K/S: 9% (14 mmol)

B. 4,5-diphenylthiazol-2-yl (comparative examples)

1. $R^4$ = carboxyphenyl

a) $R^3$ = 3,4-methylenedioxyphenyl
UV: 495 (12.6 x $10^3$, water)
nm: 630 - 680 nm; K/S: 16% (15 mmol)

b) $R^3$ = 4-methoxyphenyl
UV: 613 (8.66 x $10^3$, water)
nm: 630 - 680 nm; K/S: 17% (8 mmol)

c) $R^3$ = 2-thienyl
UV: 602 (8.22 x $10^3$, water)
nm: 635 - 685 nm; K/S: 18% (9 mmol)

d) $R^3$ = 3-thienyl
UV: 599 (11.8 x $10^3$, water)

e) $R^3$ = 4-fluorophenyl
UV: 592 (13.6 x $10^3$, water)
nm: 625 - 675 nm; K/S: 11% (33 mmol)

f) $R^3$ = 4-hydroxyphenyl
UV: 620 (11.2 x $10^3$, water)

2. $R^4$ = phenyl

a) $R^3$ = 2-thienyl
UV: 571 (9 x $10^3$, water)
nm: 640 - 690; K/S: 4% (14 mmol)

3. $R^4$ = 3-pyridyl

a) $R^3$ = 4-methoxyphenyl
UV: 589 (11.8 x $10^3$, water)
nm: 620 - 670 nm; K/S: 15% (15 mmol)

4. $R^4$ = 8-quinolyl

a) $R^3$ = phenyl
UV: 607 (14.4 x $10^3$, water)
nm: 620 - 680 nm; K/S: 21% (15 mmol)

5. $R^4$ = 4-nitronaphthyl

a) $R^3$ = 3-thienyl
UV: 696 (10.4 x $10^3$, water)
nm: 620 - 670 nm; K/S: 12% (14 mmol)

6. $R^4$ = 4-sulfophenyl

a) $R^3$ = 2-thienyl
UV: 610 (6.0 x $10^3$, water)

7. $R^4$ = 3,5-dicarboxyphenyl

a) $R^3$ = 3,4-methylenedioxyphenyl
UV: 597 (5.2 x $10^3$, water)
nm: 620 - 670 nm; K/S: 7% (14 mmol)

C. 4-phenylthiazo-2-yl

1. $R^4$ = 3,4,5-trimethoxyphenyl (comparative example)

a) 3,4-methylenedioxyphenyl
nm: 645 - 695; K/S: 28% (8.3 mmol)

2. $R^4$ = 4-carboxyphenyl

a) $R^3$= 3,4-methylenedioxyphenyl
nm: 620 - 670; K/S: 11% (8.3 mmol)

D. 4-(p-fluorophenyl)thiazol-2-yl

1. $R^4$ = 3,4,5-trimethoxyphenyl (comparative example)

a) $R^3$ = 3,4-methylenedioxyphenyl
nm: 645 - 695; K/S: 19% (8.3 mmol)

2. $R^4$ = 4-carboxyphenyl

a) $R^3$ = 3,4-methylenedioxyphenyl
nm: 625 - 675; K/S: 12% (8.3 mmol)

E. 4-phenyl-5-methylthiazol-2-yl

1. 3,4,5-trimethoxyphenyl (comparative example)

a) $R^3$ = 3,4-methylenedioxyphenyl
nm: 625 - 675; K/S: 22% (8.3 mmol)

2. 4-carboxyphenyl

a) $R^3$= 2-thienyl

nm: 600 - 650; K/S: 10% (10.3 mmol)

b) 4-methylphenyl

nm: 580 - 630; K/S: 5% (10.3 mmol)

F. 4-naphthyl-5-phenylthiazol-2-yl

1. $R^4$ = 4-carboxyphenyl

a) $R^3$ = 3,4-methylenedioxyphenyl

nm: 620 - 670; K/S: 10% (8.3 mmol)

b) $R^3$ = 2-thienyl

nm: 600 - 650; K/S: 4% (10.3 mmol)

G. 4-styryl-5-phenylthiazol-2-yl (comparative example)

1. 3,4,5-trimethoxyphenyl

a) 3,4-methylenedioxyphenyl

nm: 670 - 720; K/S: 21% (10.3 mmol)

## Claims

1. Phenyl substituted 2-thiazolyl tetrazolium salt indicators characterized by the formula:

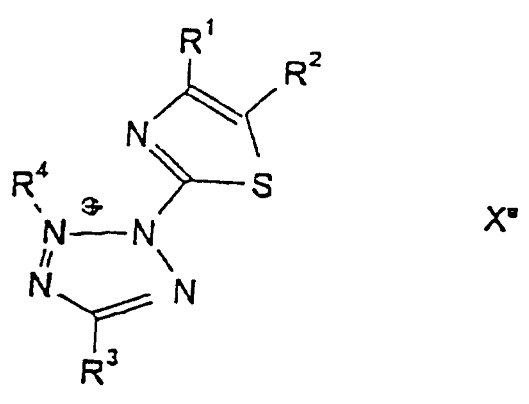

wherein $R^1$ and $R^2$ are such that the 2-thiazolyl group is 4,5-bis(4-methoxyphenyl)thiazol-2-yl, $R^4$ is 4-carboxyphenyl and $R^3$ is 2-thienyl and $X^{\ominus}$ is a counterion, and wherein the compounds are further characterized in that the reflectance spectra exhibited by each of said compounds when reduced to its colored formazan state varies by less than about 17% over a wavelength range of 50 nm when the midpoint of the 50 nm range is 640 nm or greater.

**2.** Phenyl substituted 2-thiazolyl tetrazolium salt indicators characterized by the formula:

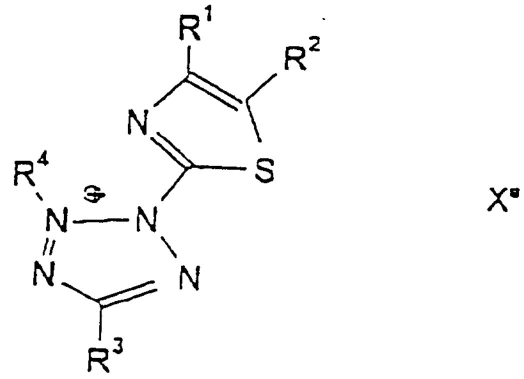

wherein $R^1$ and $R^2$ are such that the 2-thiazolyl group is 4-phenylthiazol-2-yl; $R^4$ is 4-carboxyphenyl and $R^3$ is 3,4-methylenedioxyphenyl or $R^1$ and $R^2$ are such that the 2-thiazolyl group is 4-(p-fluorophenyl)thiazol-2-yl; $R^4$ is 4-carboxyphenyl and $R^3$ is 3,4-methylenedioxyphenyl or $R^1$ and $R^2$ are such that the 2-thiazolyl group is 4-phenyl-5-methylthiazol-2-yl; $R^4$ is 4-carboxyphenyl and $R^3$ is 2-thienyl or 4-methylphenyl or $R^1$ and $R^2$ are such that the 2-thiazolyl group is 4-naphthyl-5-phenylthiazol-2-yl, $R^4$ is 4-carboxyphenyl and $R^3$ is 3,4-methylenedioxyphenyl or 2-thienyl and $X^{\ominus}$ is a counterion and wherein the compounds are further characterized in that the reflectance spectra exhibited by each of said compounds when reduced to its colored formazan state varies by less than about 17% over a wavelength range of 50 nm when the midpoint of the 50 nm range is 640 nm or greater.

**3.** 2-(4,5-Bis(4-methoxyphenyl)thiazol-2-yl)-3-(4-carboxyphenyl)-5-(2-thienyl) tetrazolium salt.

**4.** Use of the compounds of claims 1-3 for the detection of a reducing substance.

**5.** Use according to Claim 4 wherein the reducing substance is NADH

**6.** A reagent for determination of a reducing substance comprising one or more of the compounds 1 - 3.

**Patentansprüche**

**1.** Phenyl-substituierte 2-Thiazolyltetrazoliumsalz-Indikatoren, gekennzeichnet durch die Formel:

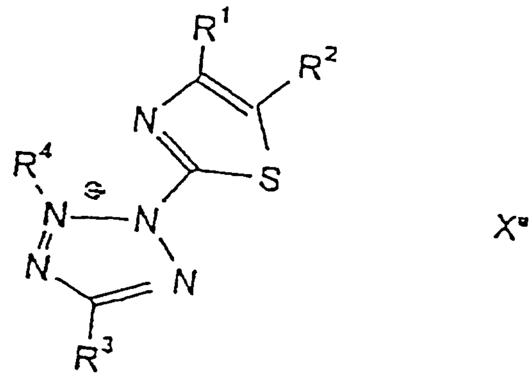

worin $R^1$ und $R^2$ so beschaffen sind, daß die 2-Thiazolylgruppe eine 4,5-Bis(4-methoxyphenylthiazol-2-yl-Gruppe ist, und worin $R^4$ eine 4-Carboxyphenyl- und $R^3$ eine 2-Thienyl-Gruppe sind, worin $X^-$ jeweils ein Gegenion ist, wobei die Verbindungen des weiteren dadurch gekennzeichnet sind, daß das Reflektionsspektrum, das jede der genannten Verbindungen bei Reduktion in ihren gefärbten Formazan-Zustand ergibt, um weniger als ca. 17% über einen Wellenlängenbereich von 50 nm schwankt, wenn der Mittelpunkt des Bereiches von 50 nm bei 640 nm oder

mehr liegt.

2. Phenyl-substiutierte 2-Thiazolyltetrazoliumsalz-Indikatoren, gekennzeichnet durch die Formel:

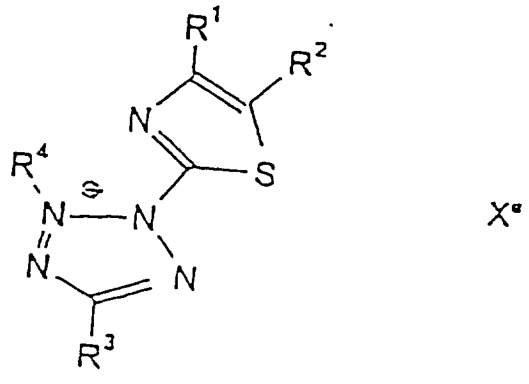

worin $R^1$ und $R^2$ so beschaffen sind, daß die 2-Thiazolyl-Gruppe eine 4-Phenylthiazol-2-yl-Gruppe ist, und worin $R^4$ eine 4-Carboxyphenyl- und $R^3$ eine 3,4-Methylendioxyphenyl-Gruppe sind, oder worin $R^1$ und $R^2$ so beschaffen sind, daß die 2-Thiazolyl-Gruppe eine 4-(p-Fluorphenyl)thiazol-2-yl-Gruppe ist, und worin $R^4$ eine 4-Carboxyphenyl- und $R^3$ eine 3,4-Methylendioxyphenyl-Gruppe sind, oder worin $R^1$ und $R^2$ so beschaffen sind, daß die 2-Thiazolyl-Gruppe eine 4-Phenyl-5-methylthiazol-2-yl-Gruppe ist, und worin $R^4$ eine 4-Carboxyphenyl- und $R^3$ eine 2-Thienyl- oder eine 4-Methylphenyl-Gruppe sind, oder worin $R^1$ und $R^2$ so beschaffen sind, daß die 2-Thiazolylgruppe eine 4-Naphthyl-5-phenylthiazol-2-yl-Gruppe ist, und worin $R^4$ eine 4-Carboxyphenyl- und $R^3$ eine 3,4-Methylendioxyphenyl- oder eine 2-Thienyl-Gruppe sind, worin $X^-$ jeweils ein Gegenion ist, wobei die Verbindungen des weiteren dadurch gekennzeichnet sind, daß das Reflektionsspektrum, das jede der genannten Verbindungen bei Reduktion in ihren gefärbten Formazan-Zustand ergibt, um weniger als ca. 17% über einen Wellenlängenbereich von 50 nm schwankt, wenn der Mittelpunkt des Bereiches von 50 nm bei 640 nm oder mehr liegt.

3. 2-(4,5-Bis(4-methoxyphenyl)thiazol-2-yl)-3-(4-carboxyphenyl)-5-(2-thienyl)tetrazoliumsalz.

4. Verwendung der Verbindungen gemäß einem der Ansprüche 1 bis 3 zum Nachweis einer reduzierenden Substanz.

5. Verwendung gemäß Anspruch 4, wobei die reduzierende Substanz NADH ist.

6. Reagens zur Bestimmung einer reduzierenden Substanz, wobei das Reagens eine oder mehrere der Verbindungen gemäß einem der Ansprüche 1 bis 3 enthält.

## Revendications

1. Indicateurs de sels de 2-thiazolyltétrazolium substitués par des phényles caractérisés par la formule

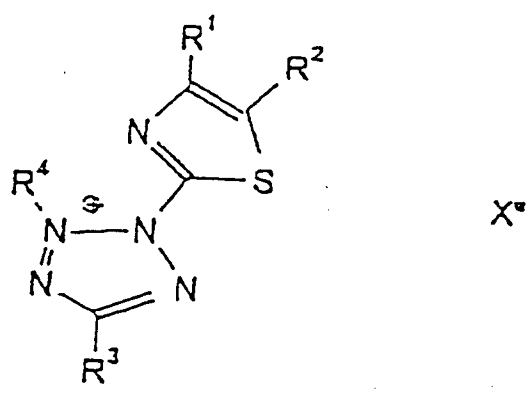

dans laquelle $R^1$ et $R^2$ sont tels que le radical 2-thiazolyle est le 4,5-bis(4-méthoxyphényl)thiazol-2-yle, $R^4$ est un radical 4-carboxyphényle et $R^3$ est un radical 2-thiényle et $X^-$ est un contre-ion et dans laquelle les composés sont en outre caractérisés en ce que le spectre de réflexion montré par chacun desdits composés quand ils sont réduits à leur état coloré de formazan varie de moins d'environ 17% sur un intervalle de longueur d'onde de 50 nm quand le point milieu de l'intervalle de 50 nm est 640 nm ou plus.

**2.** Indicateurs de sels de 2-thiazolyltétrazolium substitués par des phényles caractérisés par la formule

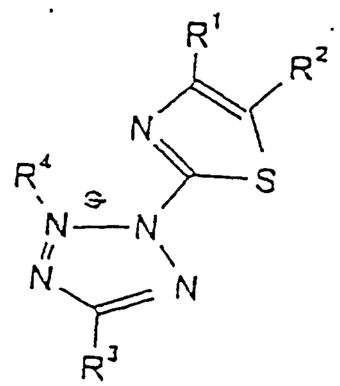

$X^-$

dans laquelle $R^1$ et $R^2$ sont tels que le radical 2-thiazolyle est le 4-phénylthiazolyle; $R^4$ est un radical 4-carboxyphényle et $R^3$ est un radical 3,4-méthylènedioxyphényle ou $R^1$ et $R^2$ sont tels que le radical 2-thiazolyle est le 4-(p-fluorophényl)thiazol-2-yle; $R^4$ est un radical 4-carboxyphényle et $R^3$ est un radical 3,4-méthylènedioxyphényle ou $R^1$ et $R^2$ sont tels que le radical 2-thiazolyle est le 4-phényl-5-méthylthiazol-2-yle; $R^4$ est un radical 4-carboxyphényle et $R^3$ est un radical 2-thiényle ou 4-méthylphényle ou $R^1$ et $R^2$ sont tels que le radical 2-thiazolyle est le 4-naphtyl-5-phénylthiazol-2-yle; $R^4$ est un radical 4-carboxyphényle et $R^3$ est un radical 3,4-méthylènedioxyphényle ou 2-thiényle et $X^-$ est un contre-ion et dans laquelle les composés sont en outre caractérisés en ce que le spectre de réflexion montré par chacun desdits composés quand ils sont réduits à leur état coloré de formazan varie de moins d'environ 17% sur un intervalle de longueur d'onde de 50 nm quand le point milieu de l'intervalle de 50 nm est 640 nm ou plus.

**3.** Sel de 2-(4,5-bis(4-méthoxyphényl)thiazol-2-yl)-3-(4-carboxyphényl)-5-(2-thiényl)tétrazolium.

**4.** Utilisation des composés des revendications 1-3 pour la détection d'une substance réductrice.

**5.** Utilisation selon la revendication 4 dans laquelle la substance réductrice est NADH.

**6.** Réactif pour la détermination d'une substance réductrice comprenant un ou plusieurs des composés des revendications 1-3.

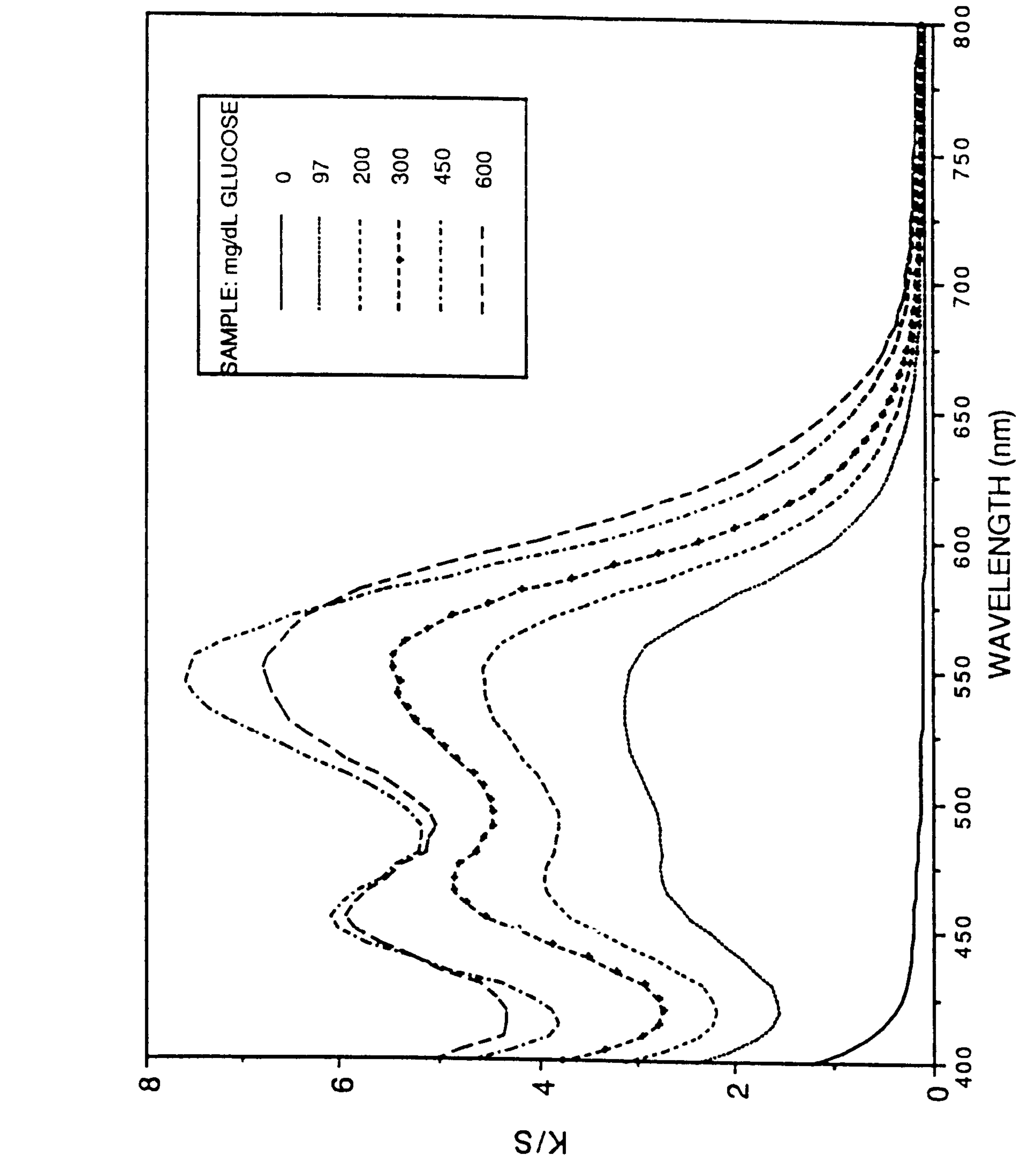
SAMPLE: mg/dL GLUCOSE
0
97
200
300
450
600
K/S
0
2
4
6
8
400
450
500
550
600
650
700
750
800
WAVELENGTH (nm)

FIG. I

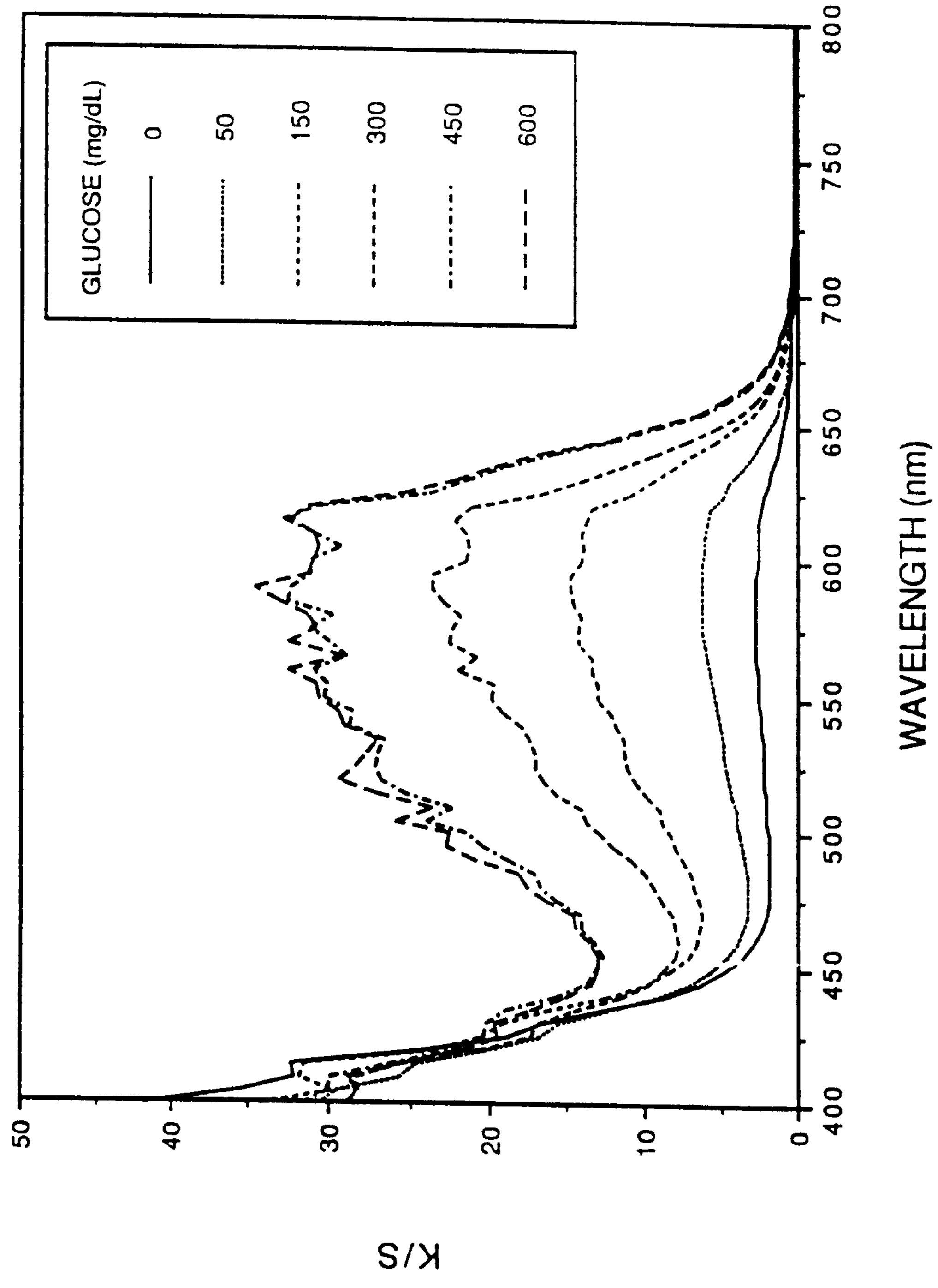
GLUCOSE (mg/dL)
0
50
150
300
450
600
K / S
0
10
20
30
40
50
400
450
500
550
600
650
700
750
800
WAVELENGTH (nm)

FIG. 2

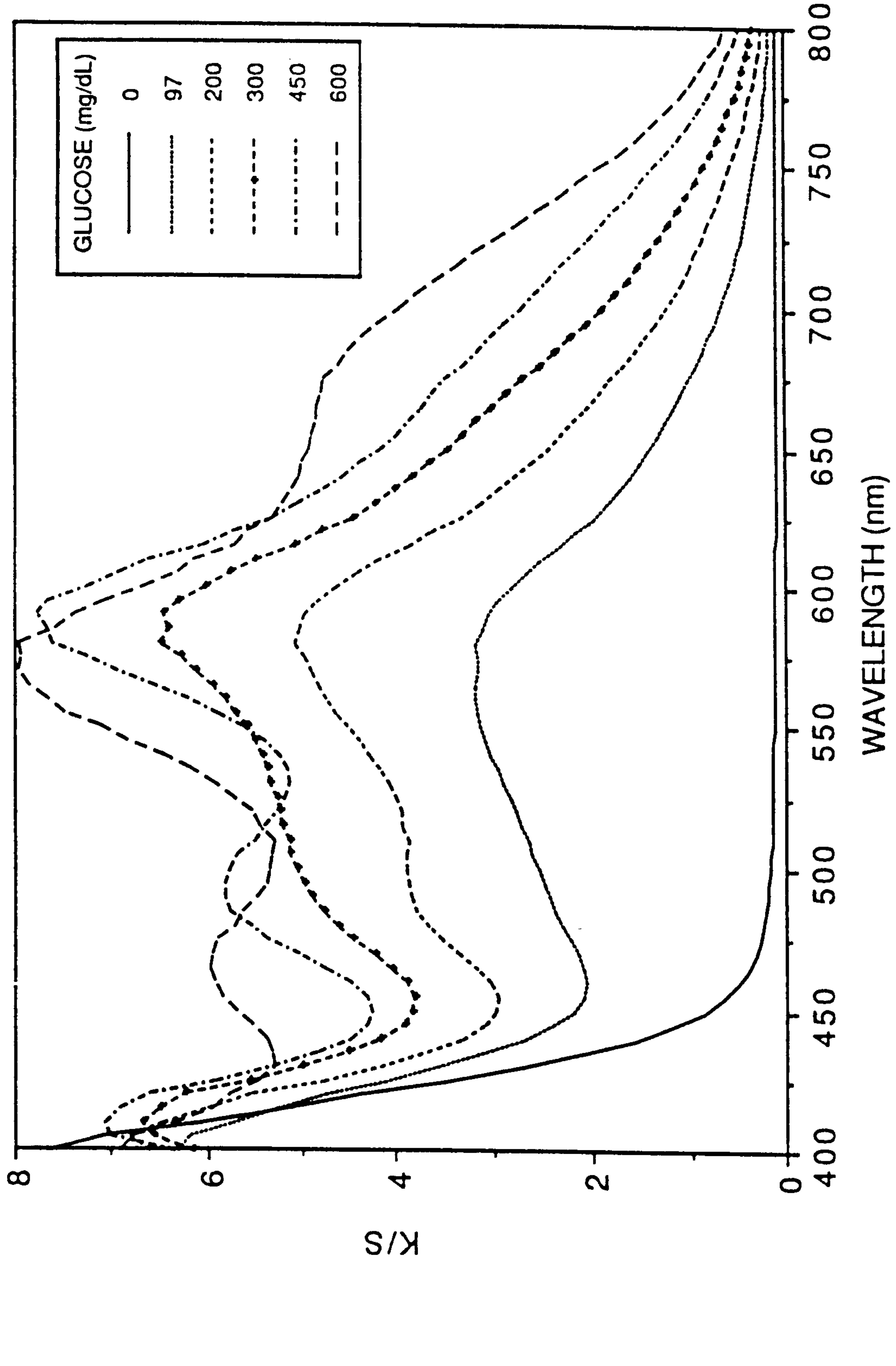
GLUCOSE (mg/dL)
0
97
200
300
450
600
K/S
8
6
4
2
0
400
450
500
550
600
650
700
750
800
WAVELENGTH (nm)

FIG. 3

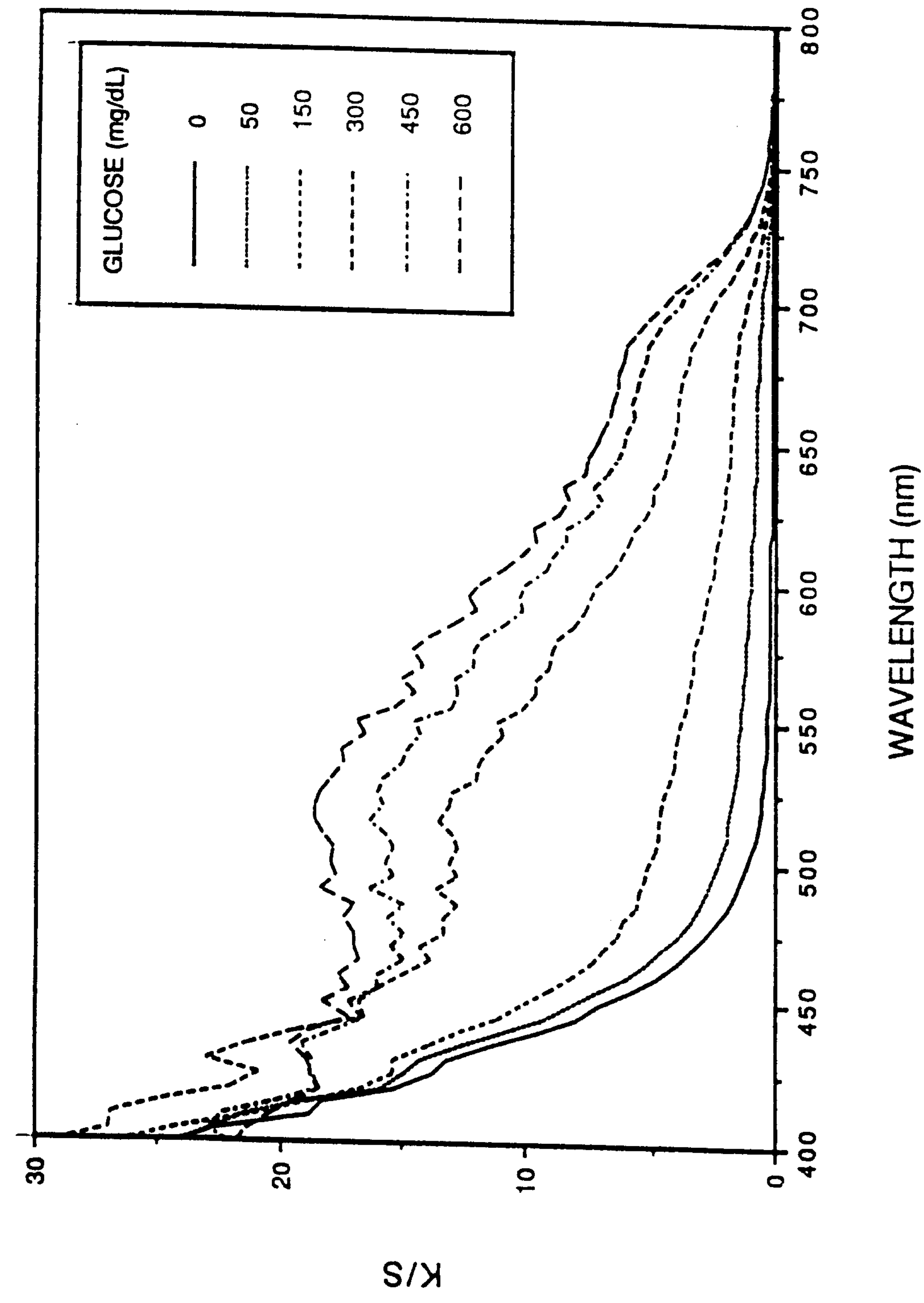
GLUCOSE (mg/dL)
0
50
150
300
450
600
K/S
30
20
10
0
400
450
500
550
600
650
700
750
800
WAVELENGTH (nm)

FIG. 4

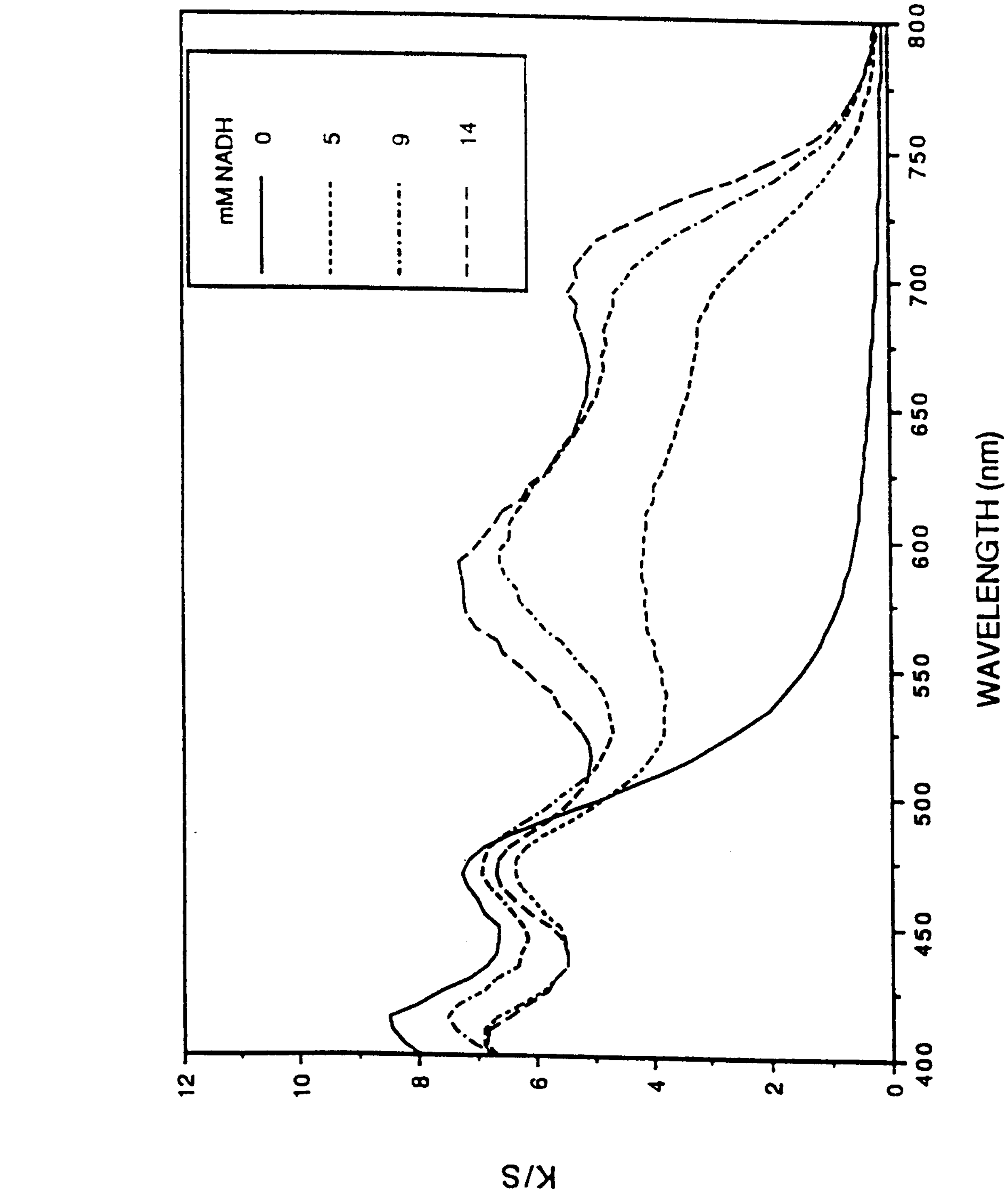
mM NADH
0
5
9
14
K/S
0
2
4
6
8
10
12
400
450
500
550
600
650
700
750
800
WAVELENGTH (nm)

FIG. 5